(19) Europäisches Patentamt
European Patent Office
Office européen des brevets

(11) Veröffentlichungsnummer: **0 443 161 B1**

(12) **EUROPÄISCHE PATENTSCHRIFT**

(45) Veröffentlichungstag der Patentschrift: **22.02.95**

(51) Int. Cl.6: **G01N 33/52**

(21) Anmeldenummer: **90124656.1**

(22) Anmeldetag: **19.12.90**

(54) **Testträger zur Analyse einer Probenflüssigkeit.**

(30) Priorität: **19.02.90 DE 4005021**

(43) Veröffentlichungstag der Anmeldung:
**28.08.91 Patentblatt 91/35**

(45) Bekanntmachung des Hinweises auf die
Patenterteilung:
**22.02.95 Patentblatt 95/08**

(84) Benannte Vertragsstaaten:
**AT BE CH DE DK ES FR GB GR IT LI LU NL SE**

(56) Entgegenhaltungen:
**EP-A- 0 182 373**
**EP-A- 0 313 858**
**DE-A- 3 130 749**
**GB-A- 2 052 057**
**US-A- 3 723 064**

(73) Patentinhaber: **BOEHRINGER MANNHEIM GMBH**

**D-68298 Mannheim (DE)**

(72) Erfinder: **Eikmeier, Heino, Dr.**
**Bismarckstrasse 8**
**W-6143 Lorsch (DE)**
Erfinder: **Rothe, Anselm, Dr.**
**Tiefenklinger Weg 21**
**W-6943 Birkenau (DE)**

(74) Vertreter: **Pfeifer, Hans-Peter, Dr.rer.nat.**
**Patentanwalt**
**Nowackanlage 15**
**D-76137 Karlsruhe (DE)**

**Beschreibung**

Die Erfindung betrifft einen Testträger zur Analyse einer Probenflüssigkeit mit mehreren Testschichten, die auf dem Testträger so angeordnet sind, daß sie von der Probenflüssigkeit nacheinander benetzt werden. Die Probe wird dabei auf eine der Schichten, die als Probenaufgabeschicht bezeichnet wird, aufgebracht. Die Testschichten bilden einen Flüssigkeitstransportweg für den Testträger.

Testträger werden für die Analyse von Probenflüssigkeiten, insbesondere Körperflüssigkeiten von Mensch oder Tier verwendet. Sie zeichnen sich gegenüber den vorbekannten Analyseverfahren mit Hilfe von flüssiger Reagenzien vor allem durch eine einfache Handhabung aus. Dadurch können die Analysen auch ohne spezialisiertes Personal und "patientennah" durchgeführt werden.

In den Testschichten ist ein meist aus mehreren Reagenzien bestehendes Reagenzsystem eingebettet, wobei die Reagenzien von der Flüssigkeit aufgelöst werden und die Reaktion von Probe und Reagenzien zu einem nachweisbaren Signal, insbesondere einer Farbänderung in einer Nachweisschicht führt. Das Nachweissignal kann visuell oder (vorzugsweise) apparativ (im Falle einer Farbänderung üblicherweise reflexionsphotometrisch) ausgewertet werden. Die Erfindung richtet sich insbesondere auf Testträger mit einem optisch meßbaren Nachweissignal.

Ein Problem bei der Durchführung komplizierter Analysen mit Hilfe von Testträgern besteht darin, daß es nicht ohne weiteres möglich ist, mehrere Reaktionsstufen zeitlich voneinander zu trennen. Die Probenflüssigkeit durchströmt den Flüssigkeitstransportweg normalerweise weitgehend ungesteuert.

Die Erfindung richtet sich auf einen Testträgertyp, bei dem mit Hilfe einer Trennschicht ein zweistufiger Verfahrensablauf möglich ist. Ein solcher Testträger ist in der DE-C-31 30 749 beschrieben. Dabei wird als Trennschicht ein hydrophobes Netz verwendet, welches in dem Flüssigkeitstransportweg zwischen der Reservoirschicht aus absorbierendem Material und der Nachweisschicht angeordnet ist. Bei dem vorbekannten Testträger liegen die Reservoirschicht, das hydrophobe Netz und die Nachweisschicht einfach übereinander auf einer streifenförmigen Basisschicht aus steifer Kunststoffolie. Sie sind an einer Kante mit der Basisschicht verklebt. Die Reservoirschicht ist größer als die darüber angeordneten Schichten, so daß sie nur teilweise von diesen bedeckt wird. Die Probe wird auf den nicht von der Nachweisschicht bedeckten Teil der Reservoirschicht aufgebracht und breitet sich in dieser unter die Nachweisschicht aus. Durch das hydrophobe Netz wird dabei sichergestellt, daß die Nachweisschicht zunächst noch nicht von der Flüssigkeit benetzt wird. In diesem Zustand kann eine erste Reaktionsstufe ablaufen, wobei die Reagenzien in der Reservoirschicht oder in weiteren Testschichten enthalten sein können, die der Reservoirschicht im Flüssigkeitstransport vorgelagert sind.

Erst wenn manuell oder mechanisch (durch ein entsprechendes Bauteil des Auswertegerätes) ein Druck auf das aus Reservoirschicht, dem hydrophoben Netz und der Nachweisschicht bestehende Schichtpaket senkrecht zur Fläche der Schichten ausgeübt wird, wird die durch das Netz gebildete Flüssigkeitsbarriere überwunden. Die Probenflüssigkeit benetzt die Nachweisschicht (und ggf. weitere zwischen dem hydrophobem Netz und der Nachweisschicht angeordnete Testschichten) und die eigentliche Nachweisreaktion wird eingeleitet.

Die Verwendung eines hydrophoben Netzes als Trennschicht erlaubt eine saubere Trennung von zwei Reaktionsstufen auf einem Testträger. Nachteilig ist aber, daß die Farbbildung in der Nachweisschicht vielfach nicht ausreichend gleichmäßig ist. Dieses Problem läßt sich zwar dadurch beseitigen, daß mit einer dicken Reservoirschicht und einer entsprechend großen Probenmenge gearbeitet wird. Zusätzlich oder alternativ kann die Struktur der Nachweisschicht so ausgebildet sein, daß sie eine gute laterale Ausbreitung der Flüssigkeit gewährleistet oder daß eine zusätzliche Ausbreitungsschicht zwischen dem hydrophoben Netz und der Nachweisschicht angeordnet wird. Diese Maßnahmen führen jedoch zu einer erhöhten Flüssigkeitsaufnahme des Testträgers. Dies steht im Gegensatz zu dem Bestreben, Testträger mit einer möglichst geringen Flüssigkeitsaufnahme zur Verfügung zu stellen, weil dadurch die notwendige Menge an Probenflüssigkeit und die im Testträger erforderliche Reagenzienmenge vermindert wird. Außerdem soll die Nachweisschicht selbst möglichst dünn sein, um eine intensive Farbbildung zu gewährleisten. Eine dünne Nachweisschicht hat aber im Regelfall eine schlechte laterale Ausbreitung und ist deswegen empfindlich gegen ungleichmäßige Farbbildung.

Der Erfindung liegt daher die Aufgabe zugrunde, einen Testträger zur Verfügung zu stellen, bei dem ein zweistufiger Reaktionsablauf möglich ist und gleichzeitig eine gleichmäßige Benetzung der Nachweisschicht selbst dann gewährleistet ist, wenn mit einer sehr kleinen Probenmenge und dementsprechend mit sehr dünnen Schichten mit geringer Flüssigkeitsaufnahme gearbeitet wird.

Die Aufgabe wird bei dem zuvor beschriebenen Testträgertyp dadurch gelöst, daß

- die Trennschicht aus einem hydrophilen Material besteht,

- die Trennschicht eine gitterförmige Struktur hat, wobei die mittlere Weite der Gitteröffnungen mindestens etwa 0,05 mm beträgt, und
- die Fäden, aus denen die gitterförmige Struktur gebildet ist, multifil sind.

Überraschenderweise hat eine derartige Trennschicht zugleich eine zuverlässige Trennwirkung und eine Verteilungswirkung für die Flüssigkeit, die eine gleichmäßige Farbbildung ermöglicht.

Multifile Fäden bestehen aus einer Vielzahl von miteinander verzwirnten Fasern. Obwohl in der zitierten DE-C-31 30 749 als geeignet für das hydrophobe Netz unter anderem Wolle und Baumwolle, also ihrer Natur nach multifile Materialien, genannt sind, wurden in der Praxis die hydrophoben Netze aus monofilen Fäden hergestellt.

Hydrophil im Sinne der vorliegenden Erfindung ist ein Faden, der die Probenflüssigkeit durch Kapillarwirkung ansaugt und in sich transportiert. Im Zweifel kann die Hydrophilität leicht durch einen Versuch festgestellt werden.

Man taucht den Faden senkrecht in eine Lösung von 7% Rinderserumalbumin (RSA) in Wasser. Bei einem hydrophoben Faden ist kein Flüssigkeitsanstieg zu beobachten, während ein hydrophiler Faden die Flüssigkeit bis zu einer Steighöhe, die für seine Hydrophilität charakteristisch ist, ansaugt. Vorzugsweise kommen bei der Erfindung verhältnismäßig schwach hydrophile Fäden zur Anwendung, wobei als Anhaltspunkt gelten kann, daß die Saughöhe in 7%iger RSA-Lösung nach einer Minute mindestens 3 mm und maximal etwa 20 mm betragen sollte.

Ein anderes Verfahren zur Testung der Hydrophilität ist in der US-A-4 292 272 beschrieben, die sich mit einer Ausbreitungsschicht aus fasrigem Material befaßt. Dort wird als Maßstab die Ausbreitung eines Tropfen einer 7%igen RSA-Lösung auf dem Schichtmaterial verwendet.

Die Weite der Gitteröffnungen muß so groß sein, daß die Probenflüssigkeit nicht durch Kapillarwirkung die Zwischenräume zwischen den Fäden der Trennschicht überspannt. In diesem Sinne werden 0,05 mm als Mindestwert angesehen, ein bevorzugter Wert beträgt etwa 0,1 mm. Auch zu große Gitteröffnungen sind von Nachteil sowohl hinsichtlich der Gleichmäßigkeit der Farbbildung als auch hinsichtlich der Trennwirkung. Die Obergrenze liegt etwa bei 0,2 mm.

Die Erfindung wird im folgenden anhand eines in den Figuren schematisch dargestellten Ausführungsbeispiels näher erläutert; es zeigen:

Fig. 1   Eine perspektivische Darstellung eines erfindungsgemäßen Testträgers,

Fig. 2   eine Seitenansicht eines erfindungsgemäßen Testträgers,

Fig. 3   eine stark vergrößerte Aufsicht auf ein für die Erfindung geeignetes Trennschichtmaterial.

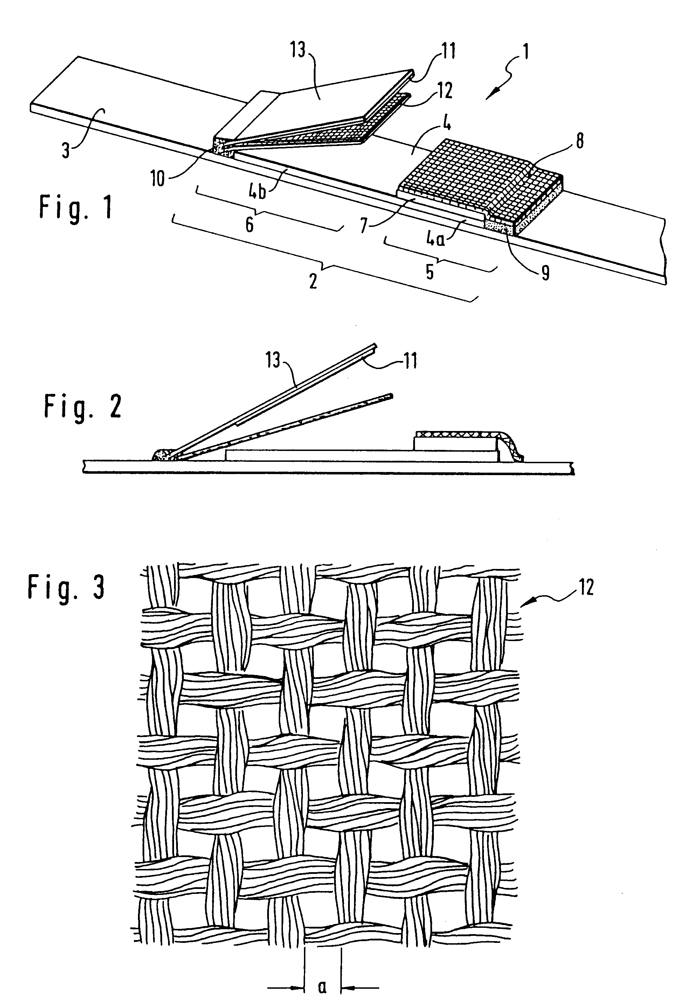

Der in Fig. 1 dargestellte Testträger 1 hat die prinzipielle Form eines konventionellen Teststreifens. Es handelt sich jedoch um ein mit früheren Teststreifen kaum vergleichbares hochwertiges Analysesystem. Andere bekannte Testträgertypen sind beispielsweise als quadratische mit einem Rahmen versehene Plättchen ausgebildet, in deren Mitte sich ein Testfeld ähnlich einem Diapositiv befindet. Die Erfindung ist auch für derartige Testträger einsetzbar, wenn ein zweistufiger Verfahrensablauf gewünscht wird.

Im Testbereich 2 des Testträgers 1 sind auf einer Basisschicht 3 aus steifer Kunststoffolie mehrere Testschichten angeordnet. Eine Reservoirschicht 4 erstreckt sich von einem Probenauftragsbereich 5 in einen Nachweisbereich 6.

In dem Probenauftragsbereich 5 ist über der Reservoirschicht 4 eine Vorreaktionsschicht 7 so angeordnet, daß sie sich über einen ersten Teilbereich 4a der Reservoirschicht 4 erstreckt. Sie ist von einem Abdecknetz 8 bedeckt, wobei die Schichten 4a, 7 und 8 an ihrer von dem Nachweisbereich abgewandten Kante mit einem Schmelzkleberstreifen 9 an der Basisschicht 3 angeklebt sind.

Im Nachweisbereich 6 sind, wiederum mit einem Schmelzkleberstreifen 10, eine Nachweisschicht 11 und eine Trennschicht 12 an der Basisschicht angeklebt. Die Verklebung ist an der von dem Probenauftragsbereich 5 abgewandten Kante der Schichten 11 und 12 so vorgesehen, daß diese im Ausgangszustand des Testträgers, also vor dessen Benutzung, schräg von der Reservoirschicht 4 abstehen. Dadurch wird ein Flüssigkeitsübertritt von der Reservoirschicht 4 in die Nachweisschicht 11 verhindert. Erst wenn die Klappe 11 manuell oder mit Hilfe eines Gerätes (wie es beispielsweise in dem US-Patent 4 780 283 beschrieben ist) nach unten gedrückt wird, wird der Flüssigkeitskontakt zwischen der Reservoirschicht 4 und der Nachweisschicht 11 hergestellt und die Probenflüssigkeit von der Nachweisschicht 11 absorbiert.

Diese Testträgerkonstruktion mit einer klappenartig abstehenden Nachweisschicht ist (beispielsweise aus der US-A-4 780 280) bekannt. Dabei ist prinzipiell ein zweistufiger Verfahrensablauf auch ohne Trennschicht 12 möglich. In der Praxis hat sich jedoch gezeigt, daß ein vorzeitiger Flüssigkeitsübertritt von der Reservoirschicht 4 in die Nachweisschicht 11 ("Frühstart") ohne Trennschicht nicht vermieden werden kann. Die Erfindung ist deswegen in Verbindung mit einem solchen Testträger von besonderer Bedeutung. Alternativ können die Trennschicht und die Nachweisschicht aber auch bereits im Ausgangszustand des Testträgers auf der Reservoirschicht aufliegen, wie dies in der DE-C 31 30 749 dargestellt ist.

Zur Durchführung einer Analyse wird ein Tropfen Probenflüssigkeit, beispielsweise Blut, auf das als Probenaufgabeschicht dienende Abdecknetz 8 aufgebracht. Die Vorreaktionsschicht 7 ist vorzugsweise als Erythrozytenabtrennschicht ausgebildet, um die roten Blutkörperchen aus dem Blut abzutrennen und reines Serum für die Analyse zur Verfügung zu stellen. Geeignet ist beispielsweise eine Glasfaserschicht gemäß US-A-4 477 575. Statt der einen Schicht 7 können im Vorreaktionsbereich auch mehrere Schichten übereinander angeordnet sein, welche verschiedene Funktionen erfüllen können, beispielsweise Reagenzien enthalten, die im späteren Verfahrensablauf benötigt werden.

Von der Schicht 7 dringt die Probenflüssigkeit in die Reservoirschicht 4 und wird in dieser lateral weitertransportiert in den Nachweisbereich 6. Der im Nachweisbereich 6 befindliche Teil 4b der Schicht 4 bildet also ein Flüssigkeitsreservoir für die nachfolgende Nachweisreaktion, welche dadurch eingeleitet wird, daß die Nachweisschicht 11 und die Reservoirschicht 4 senkrecht zu ihren Flächen gegeneinander gedrückt werden.

Für die Genauigkeit der Analyse ist es wichtig, daß eine ausreichende Menge Flüssigkeit in der Reservoirschicht 4 zur Verfügung steht. Da andererseits meist mit einem möglichst geringen Probenvolumen gearbeitet werden soll, ist es vorteilhaft, wenn die Reservoirschicht 4 eine höhere Saugkraft für die Probenflüssigkeit hat als jede im Flüssigkeitstransportweg vorgelagerte Schicht, so daß sie die vorgelagerten Schichten weitgehend "leersaugt". Im Rahmen der vorliegenden Erfindung wurde festgestellt, daß gerade solche Materialien, die eine besonders hohe Saugfähigkeit aufweisen, andererseits vielfach den Nachteil haben, daß sich die Flüssigkeit in ihnen ungleichmäßig verteilt. In solchen Fällen ist die mit der Trennschicht 12 gemäß der vorliegenden Erfindung erzielte Verteilungswirkung von besonderem Vorteil.

Die Probenflüssigkeit kann in der Reservoirschicht 4 stehenbleiben, solange dies für den Testablauf erforderlich ist. Diese Zeit kann beispielsweise vom Ablauf einer ersten Reaktionsstufe abhängig sein. Sie kann auch darauf abgestimmt sein, daß - soweit der Testträger in einer entsprechenden Apparatur temperiert wird - eine bestimmte gewünschte Temperatur erreicht ist. Danach wird zu einem festgelegten Zeitpunkt die aus den Schichten 11 und 12 bestehende Klappe nach unten gedrückt. Die Flüssigkeit tritt in die Nachweisschicht 11 über, so daß eine zweite Reaktionsstufe ablaufen kann, bei der das Nachweissignal in der Schicht 11 gebildet wird.

In Fig. 2 erkennt man einen bevorzugten Aufbau der Nachweisschicht 11, bestehend aus einer durchsichtigen Trägerfolie 13 und einem Reagenzfilm, welcher als Nachweisschicht auf die der Reservoirschicht 4 zugewandte Fläche der Folie 13 beschichtet ist. Geeignet ist insbesondere ein Reagenzfilm gemäß der US-A-4 312 834, jedoch können auch andere Filme beispielsweise auf Gelatinebasis verwendet werden. Solche Nachweisschichten sollten möglichst dünn sein, weil dadurch mit einer geringen Reagenzien- und Probenmenge eine intensive Farbbildung erreicht werden kann. Vorzugsweise ist die trockene Nachweisschicht 11 maximal 0,1 mm, besonders bevorzugt weniger als 0,05 mm dick. Ihre Flüssigkeitsaufnahme pro cm$^2$ beträgt vorzugsweise weniger als 8 $\mu$l.

Auch die Trennschicht 12 sollte dünn sein. Bevorzugt liegt ihre Dicke unter 0,15 mm, besonders bevorzugt unter 0,1 mm.

In Fig. 3 erkennt man deutlich die Struktur der bevorzugten Trennschicht 12. Die Fäden bestehen jeweils aus einer Vielzahl von miteinander verzwirnten Fasern. Sie sind zu einer gitterförmigen Struktur verbunden (im dargestellten Fall gewebt). Die in der Figur dargestellte quadratische Anordnung ist zwar bevorzugt, aber nicht notwendig. Entscheidend ist, daß die Weite a der Gitteröffnungen groß genug ist, daß diese nicht durch Kapillarwirkung von der Probenflüssigkeit vollgesaugt werden.

Ausführungsbeispiel:

Es wurden zwei verschiedene Testträger mit dem zuvor beschriebenen Aufbau hergestellt, die sich lediglich bezüglich des für die Trennschicht 12 verwendeten Materials unterscheiden.

Für das Versuchsmuster A wurde ein monofiles Nylongewebe "150 HC" von der Züricher Beuteltuchfabrik, Zürich, Schweiz verwendet. Dies ist ein aus monofilen Fäden bestehendes hydrophobes Netz gemäß der DE-C-31 30 749 und wird bei einer kommerziellen Ausführungsform von Testträgern, die eine Trennschicht auf Basis dieses Patentes aufweisen, in großem Umfang verwendet.

Bei dem Versuchsmuster B besteht die Trennschicht 12 aus einem Gewebe "PE 14/100 normal" der Schweizer Seidengazefabrik Thal, Schweiz, welches multifil und hydrophil ist.

Zur Charakterisierung der Hydrophilität wurden die Fäden beider Materialien senkrecht in eine Lösung von 7% RSA in Wasser eingetaucht. Bei dem Versuchsmuster A wurde kein Anstieg der Flüssigkeit an dem Faden beobachtet. Bei dem Versuchsmuster B stieg die Flüssigkeit in 30 sec auf 5 mm und in etwa 45 sec auf 7 mm an und blieb in dieser Höhe stehen.

EP 0 443 161 B1

Um den Einfluß auf die Meßgenauigkeit zu untersuchen, wurden mit den Versuchsmustern Blutproben mit zehn verschiedenen, bekannten Konzentrationen von Cholesterin jeweils zehnmal untersucht. Die Ergebnisse sind in der folgenden Tabelle dargestellt:

| Cholesterin im Serum (mg/dl) | Präzision Versuchsmuster A | Präzision Versuchsmuster B |
|---|---|---|
| 92 | 3,0 | 1,9 |
| 115 | 2,5 | 1,3 |
| 158 | 6,3 | 1,0 |
| 197 | 3,3 | 2,0 |
| 236 | 2,8 | 2,0 |
| 277 | 1,8 | 1,4 |
| 336 | 2,0 | 1,5 |
| 399 | 2,6 | 2,6 |
| 464 | 1,5 | 2,4 |
| 521 | 1,9 | 1,9 |
| mittlere Präzision | 2,8 | 1,8 |

Die Präzision ist jeweils (wie in der klinischen Chemie üblich) in Prozent des Variationskoeffizienten (VK) angegeben.

Es zeigt sich eine deutliche Verbesserung durch Verwendung der erfindungsgemäßen Trennschicht. Die Trennung der beiden Verfahrensstufen des Reaktionsablaufes war bei beiden Mustern gleich gut.

**Patentansprüche**

1. Testträger zur Analyse einer Probenflüssigkeit mit mehreren Testschichten, die einen Flüssigkeitstransportweg für die Probenflüssigkeit bilden und ein Reagenzsystem enthalten, dessen Reaktion mit der Probenflüssigkeit zu einem nachweisbaren Signal führt, umfassend
eine Reservoirschicht (4) aus absorbierendem Material
eine in dem Flüssigkeitstransportweg hinter der Reservoirschicht (4) angeordnete Nachweisschicht (11), in der das nachweisbare Signal gebildet wird und
eine zwischen der Reservoirschicht (4) und der Nachweisschicht (11) angeordnete Trennschicht (12), die so ausgebildet ist, daß ein Flüssigkeitskontakt zwischen der Reservoirschicht (4) und der Nachweisschicht (11) erst bei einer Druckbelastung entsteht, durch die die Reservoirschicht und die Nachweisschicht (11) gegeneinander gedrückt werden, **dadurch gekennzeichnet**, daß die Trennschicht (12) folgende Merkmale in Kombination miteinander aufweist:
sie besteht aus hydrophilem Material;
sie hat eine gitterförmige Struktur, wobei die mittlere Weite (a) der Gitteröffnungen größer als 0,05 mm ist;
die Fäden, aus denen die gitterförmige Struktur gebildet ist, sind multifil.

2. Testträger nach Anspruch 1, **dadurch gekennzeichnet,** daß die mittlere Weite (a) der Gitteröffnungen kleiner als 0,2 mm ist.

3. Testträger nach einem der vorhergehenden Ansprüche, **dadurch gekennzeichnet,** daß in dem Flüssigkeitstransportweg vor der Reservoirschicht (4) mindestens eine weitere Testschicht (7) aus absorbierendem Material vorgesehen ist und die Reservoirschicht (4) eine höhere Saugkraft für die Probenflüssigkeit als jede vor ihr in dem Flüssigkeitstransportweg angeordnete Testschicht (7) hat.

4. Testträger nach einem der vorhergehenden Ansprüche, **dadurch gekennzeichnet,** daß die Nachweisschicht (11) eine Dicke von maximal 0,1 mm hat.

5. Testträger nach einem der vorhergehenden Ansprüche, **dadurch gekennzeichnet,** daß die Nachweisschicht ein Flüssigkeitsaufnahmevolumen von maximal 8 $\mu$l pro cm$^2$ hat.

5

**6.** Testträger nach einem der vorhergehenden Ansprüche, **dadurch gekennzeichnet,** daß die Dicke der Trennschicht maximal etwa 0,15 mm beträgt.

## Claims

**1.** Test carrier for analyzing a sample fluid with several test layers which form a fluid transport path for the sample fluid and which contain a reagent system the reaction of which with the sample fluid leads to a detectable signal, comprising

a reservoir layer (4) of absorbent material,

a detection layer (11) arranged in the fluid transport path behind the reservoir layer (4), in which detection layer (11) the detectable signal is formed, and

a separating layer (12) arranged between the reservoir layer (4) and the detection layer (11), said separating layer (12) being so designed that fluid contact between the reservoir layer (4) and the detection layer (11) arises only with a pressure loading by means of which the reservoir layer (4) and the detection layer (11) are pressed against one another, **characterized in that**

the separating layer (12) exhibits the following features in combination with one another:

it consists of hydrophilic material;

it has a lattice-shaped structure, in which the mean width (a) of the lattice openings is greater than 0.05 mm;

the threads of which the lattice-shaped structure is formed are multi-filament.

**2.** Test carrier according to claim 1, **characterised in that** the mean width (a) of the lattice openings is less than 0.2 mm.

**3.** Test carrier according to any one of the preceding claims, **characterised in that** there is provided in the fluid transport path in front of the reservoir layer (4) at least one further test layer (7) of absorbent material and the reservoir layer (4) has a higher suction force for the sample fluid than any test layer (7) arranged before it in the fluid transport path.

**4.** Test carrier according to any one of the preceding claims, **characterised in that** the detection layer (11) has a maximum thickness of 0.1 mm.

**5.** Test carrier according to any one of the preceding claims, **characterised in that** the detection layer has a maximum fluid absorption volume of 8 $\mu$l per cm$^2$.

**6.** Test carrier according to any one of the preceding claims, **characterised in that** the thickness of the separating layer is not more than approximately 0.15 mm.

## Revendications

**1.** Support d'essai pour l'analyse d'un échantillon liquide comportant plusieurs couches-test qui constituent un parcours pour le transport de l'échantillon liquide et contiennent un système réactif dont la réaction avec l'échantillon liquide engendre un signal identifiable, ledit support d'essai comportant

une couche réservoir (4) en matière absorbante

une couche de contrôle (11) disposée sur le parcours du liquide en aval de la couche réservoir (4), et dans laquelle est généré le signal identifiable et une couche de séparation (12) disposée entre la couche réservoir (4) et la couche de contrôle (11), ladite couche étant conçue de façon à ce qu'un contact avec liquide ne se produise entre la couche réservoir (4) et la couche de contrôle (11) qu'en présence d'une pression qui applique la couche réservoir contre la couche de contrôle (11), **caractérisé en ce que**

la couche de séparation (12) présente les caractéristiques suivantes combinées entre elles:

elle consiste en une matière hydrophile;

elle possède une structure en forme de grille de filets dont les mailles ont une ouverture moyenne (a) supérieure à 0,05 mm;

les fils dont est composée la grille de filets sont en fils multiples.

**2.** Support d'essai selon la revendication 1, **caractérisé en ce que** l'ouverture moyenne (a) des mailles de la grille de filets est inférieure à 0,2 m.

3. Support d'essai selon l'une quelconque des revendications précédentes, **caractérisé en ce qu'**au moins une couche-test supplémentaire (7) en matière absorbante est disposée sur le parcours du liquide en amont de la couche réservoir (4) et que le pouvoir absorbant de la couche réservoir (4) par rapport à l'échantillon liquide est plus grand que celui de chacune des couches-test (7) disposées en amont de celle-ci sur le parcours du liquide.

4. Support d'essai selon l'une quelconque des revendications précédentes, **caractérisé en ce que** la couche de contrôle (11) présente une épaisseur maximale de 0,1 mm.

5. Support d'essai selon l'une quelconque des revendications précédentes, **caractérisé en ce que** la couche de contrôle possède un volume d'absorption de liquide de 8 $\mu$l par cm$^2$ au maximum.

6. Support d'essai selon l'une quelconque des revendications précédentes, **caractérisé en ce que** l'épaisseur de la couche de séparation est de l'ordre de 0,15 mm au maximum.

Fig. 1

Fig. 2

Fig. 3